# EUROPEAN PATENT SPECIFICATION

(11) **EP 1 265 982 B1**
(45) Date of publication and mention of the grant of the patent: **29.09.2004**
(21) Application number: 01904208.4
(22) Date of filing: 15.02.2001
(51) Int. Cl.: C12N 1/00

(54) **METHOD FOR THE EXTRACTION OF PROTEINS FROM A SINGLE CELL**
METHODE UM PROTEINE AUS EINEM EINZELLER ZU EXTRAHIEREN
METHODDE D'EXTRACTION DE PROTEINES D'UNE CELLULE

(30) Priority: 16.02.2000 GB 0003620
(43) Date of publication of application: 18.12.2002
(73) Proprietor: Norferm DA, 4068 Stavanger (NO)
(72) Inventor: JOHANNESSEN, Arild, N-4068 Stavanger (NO); KLEPPE, Gunnar, N-4068 Stavanger (NO); LARSEN, Jan, DK-4400 Kalundborg (DK); MOEN, Einar, N-4068 Stavanger (NO)
(74) Representative: Golding, Louise Ann
(86) International application number: PCT/GB2001/000628
(87) International publication number: WO 2001/060974

(56) References cited:
- WO-A-91/01367
- WO-A-91/02463
- FR-A- 2 311 091
- US-A- 4 678 677
- MIDDELBERG A P J: "Process-scale disruption of microorganisms" , BIOTECHNOLOGY ADVANCES,GB,ELSEVIER PUBLISHING, BARKING, VOL. 13, NR. 3, PAGE(S) 491-551 XP004045404 ISSN: 0734-9750 page 509, paragraph 2 -page 528, paragraph 1

## Description

The present invention relates to a method of treating a single-cell material to produce a product having improved functional properties. In particular, the invention relates to a method of homogenizing a single-cell material, e.g. an aqueous bacterial slurry, in which the material is subjected to a pressure drop under controlled temperature conditions.

Recently, much attention has been directed toward the development of new sources of protein which may be incorporated into foods for human and/or animal consumption. A number of different protein-containing materials have been proposed as substitutes for more traditional sources of protein, such as fish meal, soya products and blood plasma, in human foods and as animal feeds. These materials include single-cell microorganisms such as fungi, yeasts and bacteria which contain high proportions of proteins. These may be grown by single-cell reproduction and several biosynthetic processes for the production of protein through the growth of single-cell microorganisms on hydrocarbon or other substrates have been developed. Today, the most widely used protein-containing microorganisms (also referred to herein as "single-cell proteins") are those derived from fungi or yeast.

Single-cell protein materials can be used directly in foods, e.g. as a spray dried product. However, their widespread use in both human and animal food products is limited by their functional properties. For example, these have poor gelling and emulsifying properties and limited oil binding capacity.

Several methods have been proposed for improving the properties of single-cell protein materials, particularly for use in human food products. For example, US-A-3843807 (Standard Oil Company) describes a method of texturizing protein-containing single-cell microorganisms in which an aqueous yeast paste containing a mixture of both whole and broken cells is extruded. Subsequent heating and drying steps result in a product having desirable properties such as chewiness, crispness and resistance to dispersion in water, making this particularly suitable for use as an additive to human foods, e.g. in sausage and hamburger mixes. Single-cell proteins having improved functional properties have also been obtained by heat treatment of an aqueous yeast slurry (see US-A-4192897 to Standard Oil Company). The heat-treated product heightens flavour and increases smooth mouthfeel in human foods such as salad dressings, taco seasoning mix, stroganoff sauce and pizza sauce.

With the increasing use of protein-containing microorganisms as a replacement for traditional protein sources, a continuing need exists for alternative methods of improving or varying the functional properties of such materials to make these more acceptable in various food applications.

It has now been found that the functional properties of single-cell proteins can be varied and/or improved by subjecting such materials to a homogenization process, in particular to a mechanical process capable of effecting cell disruption or disintegration, for example a high pressure homogenization process in which the single-cell protein-containing material is subjected to a pressure drop. The resulting homogenized product exhibits improved functional properties, such as gel formation, water binding, oil binding and emulsifying properties when used as a nutritional protein in both human and animal foods.

Thus, according to one aspect, the present invention provides a process for imparting improved functional properties to a single-cell protein material, said process comprising the step of homogenizing an aqueous slurry of the single-cell protein. Homogenized protein material produced by such a process forms a further aspect of the invention.

Homogenization may be effected by any conventional means. Preferably, homogenization is effected using a high pressure homogenizer, for example by subjecting the single-cell material to a change in pressure, preferably a pressure drop, capable of effecting cell disintegration. Typically, the material may be subjected to a pressure drop in the range of from 40 MPa to 120 MPa (400 to 1200 bar), more preferably from 50 MPa to 110 MPa (500 to 1100 bar), e.g. from 60 MPa to 100 MPa (600 to 1000 bar). Generally, the drop in pressure will be instantaneous.

More specifically, the invention provides a process for imparting improved functional properties to a single-cell protein material, said process comprising the following steps:
(a) preparing an aqueous slurry of a single-cell material;
(b) subjecting the slurry to a pressure drop capable of effecting cell disintegration, e.g. a pressure drop in the range of from 40 MPa to 120 MPa, preferably from 50 MPa to 110 MPa, e.g. from 60 MPa to 100 MPa, whereby to produce a homogenized product; and
(c) optionally drying the homogenized product.

In a yet further aspect the invention provides a homogenized single-cell protein material or protein-containing homogenate derived from a single-cell material, preferably a homogenous bacterial biomass.

As used herein, the terms "homogenized" or "homogenate", etc. are intended to refer to any product which has been made or become homogenous, preferably a product which has been subjected to a homogenization process. The term "homogenous" is intended to encompass any substantially uniform dispersion, suspension or emulsion of cellular components. Generally speaking, any product having a degree of homogeneity of at least 60% or, more preferably, at least 70 or 80%, may be considered substantially homogenous. A substantially homogenous dispersion, suspension or emulsion may, for example, have a degree of homogeneity in excess of 90%, preferably in excess of 95%.

Typically, the homogenization process in accordance with the invention will involve treatment of microbial single-cell material in the form of a flowable aqueous paste or slurry. Generally this will consist essentially of whole cell material, although a proportion of ruptured cell material may also be present.

Unicellular organisms such as bacteria consist of a large number of extremely small cells each containing protein encapsulated within a cell-wall structure. The cell walls are relatively rigid and serve to provide mechanical support. During the homogenization process of the invention the microbial cell walls are broken whereby to release a portion of protein from within the cell structure. This may be achieved, for example, by a sequence of pressurizing and depressurizing the single-cell material. Homogenization may be effected by pressurizing the material up to a pressure of 150 MPa (1500 bars), preferably up to 140 MPa (1400 bars), e.g. up to 120 MPa (1200 bars). However, it is the actual pressure drop which is believed to determine the efficiency of the process and typical pressure drops will lie in the range of from 40 MPa to 120 MPa, more preferably from 50 MPa to 110 MPa, e.g. from 60 MPa to 100 MPa.

Typically the process will be effected in an industrial homogenizer, e.g. available from APV Rannie, Denmark, under controlled temperature conditions, preferably at a temperature of less than 50°C, particularly preferably from 25 to 50°C, e.g. from 25 to 35°C.

Other methods known in the art may be used to effect homogenization in accordance with the invention. For example, homogenization may be effected by subjecting the single-cell material to shear forces capable of disrupting the cell walls. This may be achieved using a mixer in which the material is passed through a zone in which shear forces are exerted upon it by surfaces moving relative to each other. Generally, the shear forces will be created between a moving surface, e.g. a rotating surface, and a static surface, i.e. as in a rotor-stator such as described in WO99/08782.

Other techniques known for use in methods of mechanical cell disintegration, e.g. high speed ball milling, may be used to effect homogenization. Ultrasound methods may also be used.

Any single-cell protein material may be treated in accordance with the process of the invention. However, preferred microorganisms include bacteria and yeasts. Any bacteria or yeast approved for use in food products may be used and suitable species may be readily selected by those skilled in the art. Particularly preferably, the single-cell protein material for use in the invention will be a microbial culture which consists of methanotrophic bacteria optionally in combination with one or more species of heterotrophic bacteria, especially preferably a combination of methanotrophic and heterotrophic bacteria. As used herein, the term "methanotrophic" encompasses any bacterium which utilizes methane or methanol for growth. The term "heterotrophic" is used for bacteria that utilize organic substrates other than methane or methanol for growth.

Conveniently, the single-cell material may be produced by a fermentation process in which oxygen and a suitable substrate such as a liquid or gaseous hydrocarbon, an alcohol or carbohydrate, e.g. methane, methanol or natural gas, together with a nutrient mineral solution are fed to a tubular reactor containing the microorganisms. A number of such processes are well known and described in the art.

Particularly preferred for use in the invention are single-cell protein materials derived from fermentation on hydrocarbon fractions or on natural gas. Especially preferred are single-cell proteins derived from the fermentation of natural gas. As the concentration of microorganisms increases within the fermentor, a portion of the reactor contents or broth is withdrawn and the microorganisms may be separated by techniques well known in the art, e.g. centrifugation and/or ultrafiltration. Conveniently, in such a fermentation process, the broth will be continuously withdrawn from the fermentor and will have a cell concentration between 1 and 5% by weight, e.g. about 3% by weight.

Single-cell materials produced from two or more microorganisms may be treated in accordance with the invention. Although these may be produced in the same or separate fermentors, generally these will be produced in the same fermentor under identical fermentation conditions. Materials produced from separate fermentation processes may be blended together prior to homogenization in accordance with the process of the invention.

Preferred bacteria for use in the invention include *Methylococcus capsulatus* (Bath), a thermophilic bacterium originally isolated from the hot springs in Bath, England and deposited as NCIMB 11132 at The National Collections of Industrial and Marine Bacteria, Aberdeen, Scotland. *M. capsulatus* (Bath) has optimum growth at about 45°C, although growth can occur between 37°C and 52°C. It is a gram-negative, non-motile spherical cell, usually occurring in pairs. The intracellular membranes are arranged as bundles of vesicular discs characteristic of Type I methanotrophs. *M. capsulatus* (Bath) is genetically a very stable organism without known plasmids. It can utilize methane or methanol for growth and ammonia, nitrate or molecular nitrogen as a source of nitrogen for protein synthesis.

Other bacteria suitable for use in the invention include the heterotrophic bacteria *Alcaligenes acidovorans* DB3 (strain NCIMB 12387), *Bacillus firmus* DB5 (strain NCIMB 13280) and *Bacillus brevis* DB4 (strain NCIMB 13288) which each have optimum growth at a temperature of about 45°C.

*A. acidovorans* DB3 is a gram-negative, aerobic, motile rod belonging to the family Pseudomonadaceae which can use ethanol, acetate, propionate and butyrate for growth. *B*. *brevis* DB4 is a gram-negative, endospore-forming, aerobic rod belonging to the genus *Bacillus* which can utilize acetate, D-fructose, D-mannose, ribose and D-tagatose. *B. firmus* DB5 is a gram-negative, endospore-forming, motile, aerobic rod of the genus *Bacillus* which can utilize acetate, N-acetyl-glucosamine, citrate, gluconate, D-glucose, glycerol and mannitol.

Suitable yeasts for use in the process of the invention may be selected from the group consisting of *Saccharomyces* and *Candida.*

One example of a fermentation process which uses natural gas as the sole carbon and energy source is that described in EP-A-306466 (Dansk Bioprotein). This process is based on the continuous fermentation of the methanotropic bacteria *M. capsulatus* grown on methane. Air or pure oxygen is used for oxygenation and ammonia is used as the nitrogen source. In addition to these substrates, the bacterial culture will typically require water, phosphate (e.g. as phosphoric acid) and several minerals which may include magnesium, calcium, potassium, iron, copper, zinc, manganese, nickel, cobalt and molybdenum, typically used as sulphates, chlorides or nitrates. All minerals used in the production of the single-cell material should be of food-grade quality.

Natural gas mainly consists of methane, although its composition will vary for different gas fields. Typically, natural gas may be expected to contain about 90% methane, about 5% ethane, about 2% propane and some higher hydrocarbons. During the fermentation of natural gas, methane is oxidized by methanotrophic bacteria to biomass and carbon dioxide. Methanol, formaldehyde and formic acid are metabolic intermediates. Formaldehyde and to some extent carbon dioxide are assimilated into biomass. However, methanotrophic bacteria are unable to use substrates comprising carbon-carbon bonds for growth and the remaining components of natural gas, i.e. ethane, propane and to some extent higher hydrocarbons, are oxidized by methanotrophic bacteria to produce the corresponding carboxylic acids (e.g. ethane is oxidized to acetic acid). Such products can be inhibitory to methanotrophic bacteria and it is therefore important that their concentrations remain low, preferably below 50 mg/l, during the production of the biomass. One solution to this problem is the combined use of one or more heterotrophic bacteria which are able to utilize the metabolites produced by the methanotrophic bacteria. Such bacteria are also capable of utilizing organic material released to the fermentation broth by cell lysis. This is important in order to avoid foam formation and also serves to minimize the risk of the culture being contaminated with undesirable bacteria. A combination of methanotrophic and heterotrophic bacteria results in a stable and high yielding culture.

During production of the single-cell material, the pH of the fermentation mixture will generally be regulated to between about 6 and 7, e.g. to 6.5 ± 0.3. Suitable acids/bases for pH regulation may be readily selected by those skilled in the art. Particularly suitable for use in this regard are sodium hydroxide and sulphuric acid. During fermentation the temperature within the fermentor should preferably be maintained to within the range of from 40°C to 50°C, most preferably 45°C ± 2°C.

Especially preferred for use in the invention is a microbial culture comprising a combination of the methanotrophic bacterium *Methylococcus capsulatus* (Bath) (strain NCIMB 11132), and the heterotrophic bacteria *Alcaligenes acidovorans* DB3 (strain NCIMB 12387) and *Bacillus firmus* DB 5 (strain NCIMB 13280), optionally in combination with *Bacillus brevis* DB4 (strain NCIMB 13288). The role of *A*. *acidovorans* DB3 is to utilize acetate and propionate produced by *M. capsulatus* (Bath) from ethane and propane in the natural gas. A. *acidovorans* DB3 may account for up to 10%, e.g. about 6 to 8%, of the total cell count of the resulting biomass. The role of *B*. *brevis* DB4 and *B. firmus* DB5 is to utilize lysis products and metabolites in the medium. Typically, *B. brevis* DB4 and *B. fermis* DB5 will each account for less than 1% of the cell count during continuous fermentation.

Suitable fermentors for use in preparing the single-cell material are those of the loop-type, such as those described in DK 1404/92, EP-A-418187 and EP-A-306466 of Dansk Bioprotein, or air-lift reactors. A loop-type fermentor having static mixers results in a high utilization of the gases (e.g. up to 95%) due to the plug-flow characteristics of the fermentor. Gases are introduced at several positions along the loop and remain in contact with the liquid until they are separated into the headspace at the end of the loop. Continuous fermentation may be achieved using 2-3% biomass (on a dry weight basis) and a dilution rate of 0.02 to 0.50 h⁻¹, e.g. 0.05-0.25 h⁻¹.

Other fermentors may be used in preparing the single-cell material and these include tubular and stirred tank fermentors.

Ideally, the biomass produced from fermentation of natural gas will comprise from 60 to 80% by weight crude protein; from 5 to 20% by weight crude fat; from 3 to 10% by weight ash; from 3 to 15% by weight nucleic acids (RNA and DNA); from 10 to 30 g/kg phosphorus; up to 350 mg/kg iron; and up to 120 mg/kg copper. Particularly preferably, the biomass will comprise from 68 to 73%, e.g. about 70% by weight crude protein; from 9 to 11%, e.g. about 10% by weight crude fat; from 5 to 10%, e.g. about 7% by weight ash; from 8 to 12%, e.g. about 10% by weight nucleic acids (RNA and DNA); from 10 to 25 g/kg phosphorus; up to 310 mg/kg iron; and up to 110 mg/kg copper. The amino acid profile of the protein content should be nutritionally favourable with a high proportion of the more important amino acids cysteine, methionine, threonine, lysine, tryptophan and arginine. Typically these may be present in amounts of about 0.7%, 3.1%, 5.2%, 7.2%, 2.5% and 6.9%, respectively (expressed as a per cent of the total amount of amino acids). Generally the fatty acids will comprise mainly the saturated palmitic acid (approx. 50%) and the monounsaturated palmitoleic acid (approx. 36%). The mineral content of the product will typically comprise high amounts of phosphorus (about 1.5% by weight), potassium (about 0.8% by weight) and magnesium (about 0.2% by weight).

Generally, single-cell protein materials obtained from a continous fermentation process will be subjected to centrifugation and filtration, e.g. ultrafiltration, processes to remove most of the water present and to form an aqueous paste or slurry prior to homogenization. During centrifugation the dry matter content of the biomass is typically increased from about 2 to about 15% by weight, e.g. to about 12% by weight. Ultrafiltration, which may be effected at a temperature of between 40 and 50°C, e.g. between 42 and 46°C, further concentrates the biomass to a product containing from 10 to 30%, preferably from 15 to 25%, e.g. from 15 to 22% by weight single-cell material. The size exclusion used during ultrafiltration will generally be in the range of about 100,000 Daltons.

Following ultrafiltration the biomass may be cooled, preferably to a temperature of from 10 to 30°C, e.g. to about 15°C, for example by passing the concentrated protein slurry from the ultrafiltration unit over a heat exchanger after which it may be held in a buffertank at constant temperature, e.g. for a period of from 1 to 24 hours, preferably 5 to 15 hours, e.g. 5 to 12 hours, at a temperature of from 10 to 20°C, more preferably from 5 to 15°C at a pH in the range of from 5.5 to 6.5.

Homogenization may be carried out in a conventional high pressure homogenizer in which the cells may be ruptured by first pressurizing, e.g. up to a pressure of 150 MPa (1500 bars), and then depressurizing the inside of the homogenizer. Preferably, the total pressure drop applied to the biomass will be in the range of from 40 MPa to 120 MPa (400 to 1200 bar), e.g. about 80 MPa (800 bar). The drop in pressure may be stepped, i.e. this may comprise one or more steps, although generally this will comprise one or two steps, preferably a single step. In cases where homogenization is effected as a two-step process it is preferable that the pressure drop in the second step should represent less than 1/5, preferably less than 1/10, e.g. about 1/20 of the total pressure drop in the homogenizer. The temperature of the material during homogenization should preferably not exceed 50°C.

The homogenization process herein described results in the production of a product comprising, preferably consisting essentially of, ruptured cell material. For example, ruptured cell material will be present in an amount of at least 80%, preferably at least 90% by weight. Typically, the product will be a relatively viscous protein slurry containing soluble and particulate cellular components. Although this may be used directly as an additive in food products, this will usually be further processed whereby to remove excess water from the product. The choice of any additional drying step or steps will depend on the water content of the product following homogenization and the desired moisture content of the final product.

Typically, the product will be further processed in accordance with spray drying techniques well known in the art. Any conventional spray drier with or without fluid bed units may be used, for example the Type 3-SPD spray drier available from APV Anhydro, Denmark. Preferably the inlet temperature for the air in the spray drier may be about 300°C and the outlet temperature may be about 90°C. Preferably the resulting product will have a water content of from about 2 to 10% by weight, e.g. from 6 to 8% by weight. The resulting product will typically be of a particle size of from 0.1 to 0.5mm.

Particularly preferably, the step of homogenization will be immediately followed by spray drying. Alternatively, it may be necessary, or indeed desirable, to store or hold the homogenized product, e.g. in a storage or buffer tank, prior to further processing. In such cases, it has been found that the conditions under which the product is stored may reduce the gelling properties of the final product following spray drying. The gelling properties of the homogenized material may be maintained by storing this at a temperature of less than 20°C and at a pH < 7, preferably < 6.5, particularly preferably at a pH in the range 5.5 to 6.5, e.g. 5.8 to 6.5. Under these conditions, the product may be stored for up to 24 hours without any substantial loss of gelling properties.

In a yet further aspect the invention thus provides a method of maintaining the functional properties of a homogenized single-cell protein material, said method comprising the step of storing said material at a temperature of less than 20°C and at a pH of less than about 6.5.

Products produced by the process of the invention have highly desirable properties making these particularly suitable for use in both human and animal food products, either alone or when used in combination with other protein sources. In particular, in contrast to the original single-cell protein material, the homogenized product exhibits improved functional properties such as gel formation, water binding, oil binding and emulsifying properties. For example, the product of the invention exhibits improved gel formation when mixed with conventional food grade products, in particular polysaccharides such as alginates and/or karragenates. In addition, improved oil binding capacity is observed on mixing the product with oils such as soya oil or fish oil. Typically, a 15% (w/w) solution of a homogenized product produced in accordance with the invention may exhibit a gel strength (elastic modulus) in the range of from 1000 to 2000 Pa, e.g. about 1500 Pa.

In a yet further aspect the invention thus provides the use of a homogenized single-cell protein material as herein described as a gelling agent, an emulsifier or as an oil or water binder in the preparation of a food or feed product.

The product of the invention is especially useful as a functional protein in food products, particularly when used as a substitute for natural plasma in animal feeds and in pet foods. When used in pet foods, additional ingredients may be added to the product such as fats, sugars, salt, flavourings, minerals, etc. The product may then be formed into chunks resembling natural meat chunks in appearance and texture. The product of the invention has the further advantages that this is readily formulated to contain necessary nutrients, is easily digested by the animals and is palatable to the animals.

The product of the invention may find further use as a texturant in meat products (e.g. meat balls), as a replacement for plasma proteins conventionally used as extenders in fresh meat to increase weight and volume, as an emulsifer (e.g. in dressings, etc.), and in bakery products to enhance dough properties.

When used in food products, the homogenized material will typically be used in an amount of from 1 to 10% by weight, preferably up to 5% by weight. The exact proportion will depend on the desired function of the material and can be readily determined by those skilled in the art. Typically, when used as a gelling agent this may be present in an amount of up to 20% by weight, e.g. 5 to 10% by weight (based on dry matter content of the product).

In a further aspect the invention thus provides a food grade product or additive, e.g. an animal feed or pet food, comprising a homogenized single-cell material as herein described.

In a yet further aspect the invention provides a pet food comprising chunks of a homogenized single-cell material as herein described.

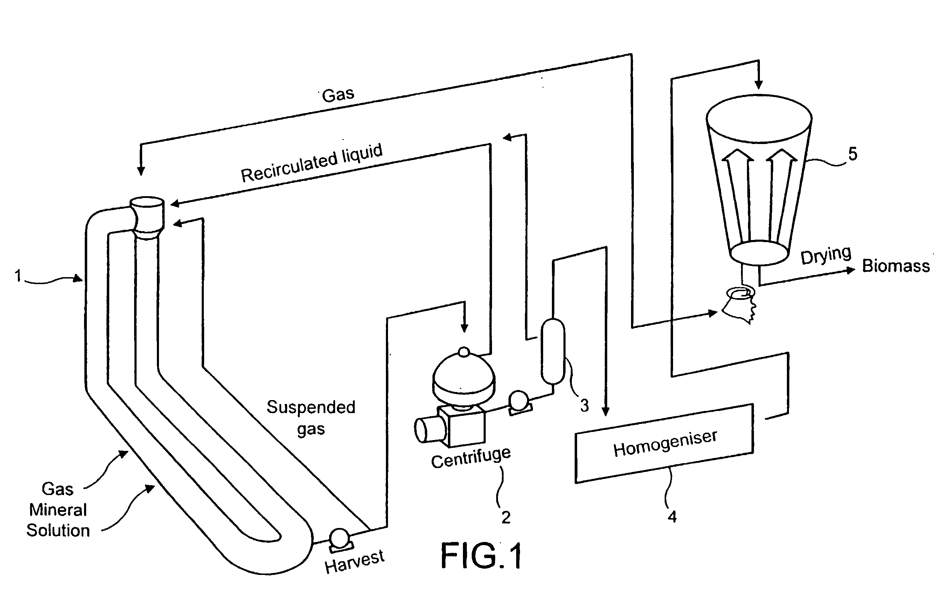

A preferred embodiment of the invention will now be described with reference to accompanying Figure 1 which schematically illustrates apparatus for use in preparing a homogenized single-cell material in accordance with the invention.

Referring to Figure 1, *Methylococcus capsulatus* (Bath) NCIMB 11132 is produced in the fermentor 1 by continuous fermentation of natural gas in an ammonium/mineral salts medium (AMS) at 45°C, pH 6.5, and at a dilution rate of 0.15 h⁻¹. The AMS medium contains the following per litre: 10 mg NH₃, 75 mg H₃PO₄.2H₂O, 380 mg MgSO₄.7H₂O, 100 mg CaCl₂.2H₂O, 200 mg K₂SO₄, 75 mg FeSO₄.7H₂O, 1.0 mg CuSO₄.5H₂O, 0.96 mg ZnSO₄.7H₂O, 120 µg CoCl₂.6H₂O, 48 µg MnCl₂.4H₂O, 36 µg H₃BO₃, 24 µg NiCl₂.6H₂O and 1.20 µg NaMoO₄.2H₂O. Oxygen is the limiting factor.

The fermentor is filled with water which has been heat-sterilized at 125°C for 10 sees. Addition of the different nutrients is regulated according to their consumption. Continuous fermentation is operated with 2-3% biomass (on a dry weight basis) .

The resulting single-cell material is continuously harvested. This is then subjected to centrifugation in centrifuge 2 in which the dry matter content is increased from about 2 to 15% by weight. The resulting aqueous biomass is transferred to a storage tank (not shown) where it is kept, e.g. at a temperature of between 5 and 15°C.

The bacterial biomass is further concentrated to a product containing from 15 to 22% by weight biomass using an ultrafiltration unit 3 having an exclusion size of 100,000 Daltons. The temperature during ultrafiltration is typically maintained between 40 and 50°C. Following ultrafiltration the biomass is cooled, e.g. to a temperature of from 10 to 30°C, by passing the concentrated protein slurry from the ultrafiltration unit over a heat exchanger (not shown). Thereafter, the protein slurry may be kept in a buffertank (not shown) at constant temperature. The resulting product is a stable culture free from contamination by undesirable bacteria.

In order to minimize the use of water and to minimize the amount of waste water, water from the centrifuge 2 and/or ultrafiltration unit 3 is returned to the fermentor after a short heat treatment.

Homogenization of the bacterial biomass is carried out in an industrial high pressure homogenizer 4. The protein slurry from the buffertank is continuously pumped through the homogenizer where the cells are broken through the pressurizing and immediate release of pressure inside the homogenizer. The total pressure drop within the homogenizer will typically vary from 60 MPa to 100 MPa (600 to 1000 bar). During homogenization the temperature is preferably maintained below 50°C.

Following homogenization, the relatively viscous protein slurry containing soluble and particulate cellular components is spray dried in spray drier 5. Spray drying at an inlet air temperature of about 300°C and an outlet temperature of about 90°C yields a final product having a water content of from 6 to 8% by weight.

The resulting homogenized protein forms distinct gels upon heating to temperatures above 60°C and subsequent cooling to ambient temperature at protein concentrations ranging between 7.5 and 15% by weight. Suitable conditions for gel formation include a pH range between 5.7 and 7.0 in which the pH is controlled using a buffer such as a phosphate or citrate buffer at concentrations ranging from 50 to 200 mmol.

Improved gel formation is observed when the homogenized product is mixed with polysaccharides such as starch, alginate and iota- and kappa carrageenan. Addition of from 0.5 to 1.0% by weight carrageenan to the product (7.5 to 15%) in an aqueous solution of 0.1 to 2.0% potassium chloride at a pH ranging from 5 to 7.5 greatly enhances the gel strength, indicating synergistic interaction between the product and polysaccharides. Similar synergistic effects on rheology and gel formation have been observed on mixing the product with various starch sources (in amounts of from 3 to 10% final starch concentration) such as purified starch and chemically modified starch containing various amounts of amylose and amylopectin.

Emulsifying properties of the homogenized product are demonstrated by mixing the protein (2 to 4%) in a sodium chloride solution at a pH of from 5 to 7 with soya oil (20 to 40% final concentration). This solution is mixed in a thurrax blender for 20 secs prior to preparation of the emulsion in a microfluidizer at a pressure of 70 MPa (700 bars) with the emulsion recycled through the microfluidizer four times. Emulsions produced from the homogenized product may be further stabilized by the addition of xanthan (e.g. in an amount of from 0.1 to 0.5% by weight) prior to mixing of the starting materials.

Oil binding capacity of the homogenized material is demonstrated by mixing the product with oils such as soya oil or fish oil in a slurry, e.g. by mixing 0.5g of the homogenized product in 3 ml soya oil followed by incubation for 30 mins at ambient temperature. Due to oil adsorption, the weight of the product can be expected to increase by 50 to 80%.

The following non-limiting examples serve to further illustrate the invention.

### Example 1 - Preparation of homogenized biomass

A microbial culture comprising *Methylococcus capsulatus* (Bath) (strain NCIMB 11132), *Alcaligenes acidovorans* DB3 (strain NCIMB 12387) and *Bacillus firmus* DB 5 (strain NCIMB 13280), is produced in a loop-type fermentor by continuous aerobic fermentation of natural gas in an ammonium/mineral salts medium (AMS) at 45°C, pH 6.5, and at a dilution rate of 0.15 h⁻¹. The AMS medium contains the following per litre: 10 mg NH₃, 75 mg H₃PO₄.2H₂O, 380 mg MgSO₄.7H₂O, 100 mg CaCl₂.2H₂O, 200 mg K₂SO₄, 75 mg FeSO₄.7H₂O, 1.0 mg CuSO₄.5H₂O, 0.96 mg ZnSO₄.7H₂O, 120 µg CoCl₂.6H₂O, 48 µg MnCl₂.4H₂O, 36 µg H₃BO₃, 24 µg NiCl₂.6H₂O and 1.20 µg NaMoO₄.2H₂O.

The fermentor is filled with water which has been heat-sterilized at 125°C for 10 secs. Addition of the different nutrients is regulated according to their consumption. Continuous fermentation is operated with 2-3% biomass (on a dry weight basis).

A single-cell material having the following characteristics is continuously harvested:

| | | | |
|---|---|---|---|
| **Composition (% in product)** | | **Minerals** | |
| Crude protein* | 66 | Phosphorus | 1.0% |
| Crude fat | 9 | Chlorine | 0.7% |
| Ash | 7 | Sulphur | 0.5% |
| Water | 6 | Calcium | 0.4% |
| Crude fibre | 1 | Potassium | 0.4% |
| N-free extract | 11 | Magnesium | 0.2% |
| **Total** | **100** | Sodium | 0.1% |
| | | Iron | 200 ppm |
| **Amino Acids (% in product)** | | Copper | 90 ppm |
| Lysine | 4.3 | Zinc | 15 ppm |
| Methionine | 1.9 | Arsenic | 0.05 ppm |
| Cystine | 0.4 | Selenium | 0.02 ppm |
| Threonine | 3.1 | Lead | 0.0002 ppm |
| Tryptophan | 1.5 | Cadmium | 0.00002 ppm |
| Leucine | 5.2 | Mercury | <0.02 ppm |
| Isoleucine | 3.2 | | |
| Valine | 4.2 | **Vitamins** | **mg/kg** |
| Tyrosine | 2.8 | Nicotine acid 123 | |
| Phenylalanine | 3.1 | Riboflavin B2 | 69 |
| Histidine | 1.7 | Inositol | 28 |
| Arginine | 4.1 | Thiamin Bl | 11 |
| Alanine | 4.9 | | |
| Aspartic Acid | 6.2 | **Energy** | **MJ/kg** |
| Glutamic Acid | 7.3 | Gross energy | 22.1 |
| Glycine | 3.4 | | |
| Proline | 3.0 | **Other Data** | |
| Serine | 2.5 | Colour | Light brown |
| **Total** | **62.8** | Flavour | Neutral |
| | | Particle Size | 100-300 ppm |

| | | | |
|---|---|---|---|
| * on dry weight basis the crude protein content is approx. 70%. | | | |

The biomass is subjected to centrifugation in an industrial continuous centrifuge at 3,000 rpm, followed by ultrafiltration using membranes having an exclusion size of 100,000 Daltons. The resulting product is then subjected to homogenization in an industrial homogenizer (pressure drop: 1000 bar (100 MPa); inlet temperature: 15°C) to produce a homogenized biomass in accordance with the invention.

### Example 2 - Properties of homogenized biomass

### 2.1 Coagulation/Gelation

Coagulation can be measured as an increase in elastic modulus (G') as the protein becomes heat denatured. This may be done in a Paar Physica UDS200 rheometer using a 15% (w/w) solution of the homogenized biomass in 0.2M Na-phosphate, pH 7.2. The instrument measures G' at a constant strain (1 x 10⁻³ Pa) and frequency (1 Hz) whilst the temperature is varied as follows:

| | |
|---|---|
| 20 to 90°C | 10 mins |
| 90°C | 5 mins |
| 90 to 20°C | 5 mins |
| 20°C | 2 mins |

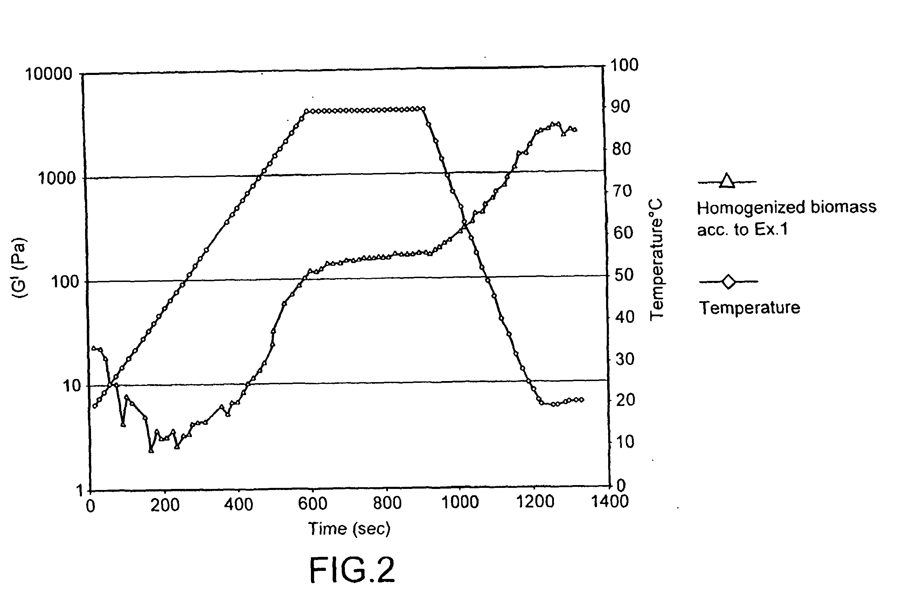

Results for the product produced in Example 1 are shown in attached Figure 2. Coagulation of the homogenized biomass starts at a temperature between 60 and 70°C. A 10 to 20-fold increase in elastic modulus is observed as the temperature is increased to 90°C. Only a minor increase in elastic modulus is observed whilst the temperature is maintained at 90°C. Cooling to 20°C results in a further 10-fold increase in elastic modulus.

### 2.2 Emulsification

### 2.2.1 Without stabilisation

The homogenized biomass prepared in Example 1 was suspended in 1.5% NaCl and mixed vigorously to break the particles. pH was adjusted as desired (e.g. to pH 6.5) and the solution stirred for 1 hour. The solution was combined with soya-oil and an emulsion was made using a Ytron-MS mixer type MSU.G.C.AA at full speed for 3 mins. The emulsion was transferred to a test tube and the separation of oil or water recorded. For those emulsions which were heated, the test tubes were submerged into a boiling water bath for 30 mins.

The homogenized biomass of Example 1 results in stable emulsions which do not separate an oil phase. However, creaming may occur in low viscous systems.

### 2.2.2 With stabilisation

Xanthan was used as stabilising agent. The xanthan was dissolved in 1.5% NaCl by stirring at ambient temperature overnight. The homogenized biomass emulsions were then prepared with both 0.5% and 0.25% xanthan as described in 2.2.1.

Increasing the viscosity using xanthan gum results in emulsions without creaming and which remain stable for several weeks. Heating does not break the emulsion. However, coagulation of homogenized biomass occurs and separation of water between coagulates may be observed. The coagulation pattern can be disrupted for most emulsions by gentle mixing, thereby maintaining a stable emulsion.

Stable homogenized biomass emulsions can be produced at different pH. Acid pH combined with heat results in some creaming, most probably due to hydrolysis of xanthan, and thus lowered viscosity.

Homogenized biomass in accordance with the invention may produce oil-in-water emulsions containing up to 80% oil. Increasing the oil concentration to 85% inverts the emulsion resulting in water-in-oil emulsions.

### 2.3 Fat/Oil absorption

Fat absorption was measured by suspending 0.5g of the homogenized biomass of Example 1 in 3 ml soya oil followed by incubation for >30 mins at ambient temperature. The suspensions were filtered on a <10µm filter and the amount of oil (in g) absorbed to the oil insoluble material was measured.

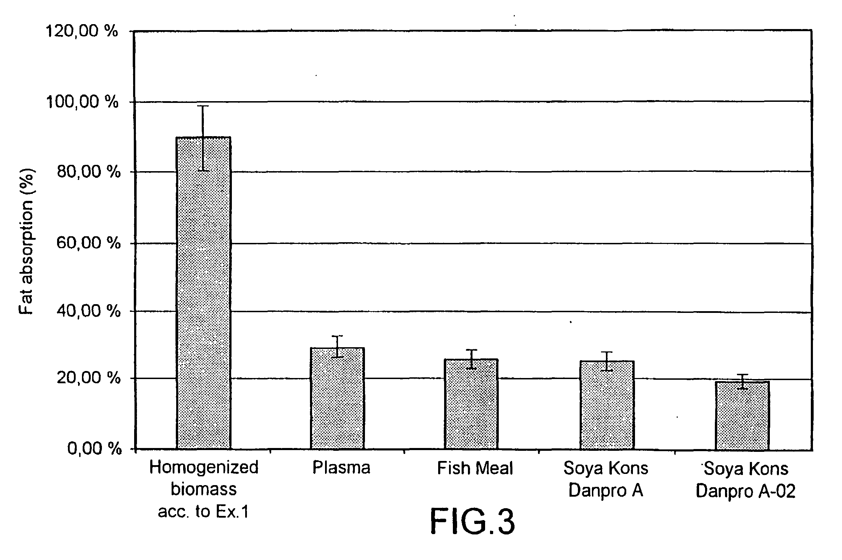

Fat/Oil absorption measured for the homogenized biomass of Example 1 and for several commercially available proteins* is shown in attached Figure 3. As may be seen, the product in accordance with the invention binds fat more efficiently than the other commercial proteins tested.
(* "Plasma" = AP 820 spray-dried animal plasma from APC Europe;
"Fish Meal" = LT 3012 Fish Meal from Nordsildmel A/S; and
"Soya Kons Danpro A" and "Soya Kons Danpro A-02" = soya concentrates from Central Soya Aarhus A/S).

### 2.4 Water absorption

A 2.5% suspension of the homogenized product of Example 1 was made and mixed vigorously to disrupt the particles. pH was adjusted to 4, 6 and 8 using 1M NaOH or 1M HCl and the suspension stirred for a further 60 mins at ambient temperature. The resulting suspensions were then centrifuged in a SS-34 rotor at 10.000 x g for 30 mins.

The supernatant was poured off and excess water was removed by turning the tube upside down for 10 mins. The weight of the wet pellet was measured and then dried overnight at 105°C. The weight of the dry pellet was then measured.

Water absorption is measured either as the amount of water (in g) absorbed by the water insoluble pellet (water binding) or as water absorbed (in g) to total protein (water imbibing). Results are shown in Table 1 below:

**Table 1 -**

| Water absorption | | | |
|---|---|---|---|
| | pH | Water binding (g) | Water imbibing (g) |
| Homogenized biomass according to Ex. 1 | 4.02 | 3.23 | 2.40 |
| | 6.30 | 3.99 | 2.06 |
| | 7.90 | 5.60 | 2.07 |
| AP 820 Spray-dried animal plasma from APC Europe | 4.03 | 7.64 | 0.69 |
| | 6.05 | 7.15 | 0.64 |
| | 7.53 | 10.11 | 0.94 |
| LT 3012 Fish Meal from Nordsildmel A/S | 4.48 | 2.95 | 1.71 |
| | 6.42 | 3.12 | 1.93 |
| | 8.03 | 3.90 | 2.55 |

From Table 1 it can be seen that the insoluble part of the homogenized biomass in accordance with the invention absorbs about 4-6 times its weight in water and the protein part about 2-2.5 times its weight in water.

### 2.5 Solubility

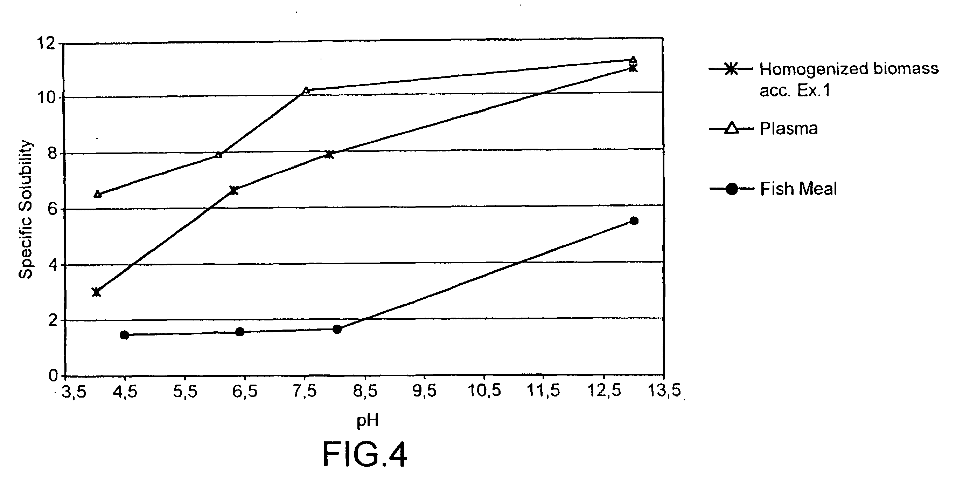

Protein solubility can be determined in similar experiments as for water absorption, but instead measuring the soluble protein in the supernatant. Results for the homogenized biomass of Example 1 and for various commercially available proteins* are shown in attached Figure 4.
(* "Plasma" = AP 820 spray-dried animal plasma from APC Europe; and "Fish Meal" = LT 3012 Fish Meal from Nordsildmel A/S)

The results show that the solubility of the product in accordance with the invention is dependent on pH: acid pH results in low solubility; and alkaline pH results in in high solubility. In weak acid solutions, the product of the invention behaves as a colloid particle and it is these colloidal properties which predominate.

## Claims

1. A process for homogenizing a single-cell protein material, said process comprising the step of homogenizing an aqueous slurry of the single-cell protein by subjecting it to a total pressure drop in the range of from 40 MPa to 120 MPa (400 to 1200 bar) , wherein said single-cell protein material is a microbial culture comprising methanotrophic bacteria optionally in combination with one or more species of heterotrophic bacteria.

2. A process as claimed in claim 1 further comprising the step of drying the resulting homogenized product.

3. A process as claimed in claim 1 for imparting improved functional properties to a single-cell protein material, said process comprising the following steps:
(a) preparing an aqueous slurry of a single-cell material which is a microbial culture comprising methanotrophic bacteria optionally in combination with one or more species of heterotrophic bacteria;
(b) subjecting the slurry to a total pressure drop of from 40 MPa to 120 MPa (400 to 1200 bar), whereby to produce a homogenized product; and
(c) optionally drying the homogenized product.

4. A process as claimed in any one of claims 1 to 3 wherein homogenization is effected at a temperature of less than 50°C, preferably from 25 to 50°C, e.g. from 25 to 35°C.

5. A process as claimed in any one of claims 1 to 4 wherein said pressure drop is stepped.

6. A process as claimed in claim 5 wherein said pressure drop is effected as a two-step process comprising first and second steps in which the pressure drop in the second step represents less than one fifth of the total pressure drop.

7. A process as claimed in any preceding claim wherein the resulting homogenized product is dried by spray drying.

8. A process as claimed in any preceding claim wherein prior to spray drying the homogenized product is held in a storage tank at a temperature of less than 20°C and a pH of less than about 6.5.

9. A process as claimed in any preceding claim wherein said microbial culture comprises methanotrophic and heterotrophic bacteria.

10. A process as claimed in claim 9 wherein said microbial culture comprises a combination of the methanotrophic bacterium *Methylococcus capsulatus* (Bath) (strain NCIMB 11132), and the heterotrophic bacteria *Alcaligenes acidovorans* DB3 (strain NCIMB 13287) and *Bacillus firmus* DB 5 (strain NCIMB 13289), optionally in combination with *Bacillus brevis* DB4 (strain NCIMB 13288) .

11. A process as claimed in any preceding claim wherein said single-cell material is derived from fermentation on hydrocarbon fractions or on natural gas..

12. A homogenized single-cell protein material obtainable by a process as claimed in claim 9 or claim 10.

13. A homogenized single-cell protein material comprising a combination of the methanotrophic bacterium *Methylococcus capsulatus* (Bath) (strain NCIMB 11132), and the heterotrophic bacteria *Alcaligenes acidovorans* DB3 (strain NCIMB 13287) and *Bacillus firmus* DB 5 (strain NCIMB 13289), optionally in combination with *Bacillus brevis* DB4 (strain NCIMB 13288).

14. A food grade product or additive, e.g. an animal feed or pet food, comprising a single-cell material as claimed in claim 12 or claim 13.

15. A pet food comprising chunks of a single-cell material as claimed in elaim 12 or claim 13.

16. A good grade product or additive, e.g. an animal feed or pet food, comprising a homogenized single-cell protein material obtainable by a process as claimed in any one of claims 1 to 11, together with a food grade product.

17. Use of a homogenized single-cell protein material obtainable by a process as claimed in any one of claims 1 to 11 as a gelling agent, an emulsifier or as an oil or water binder in the preparation of a food or feed product.

## Patentansprüche

1. Verfahren zur Homogenisierung eines Einzellerproteinmaterials, wobei das besagte Verfahren den Schritt der Homogenisierung eines wässerigen Schlamms des Einzellerproteins umfasst, indem es einem Gesamtdruckabfall in dem Bereich von 40 MPa bis 120 MPa (400 bis 1200 bar) unterworfen wird, worin das besagte Einzellerproteinmaterial eine mikrobielle Kultur ist, umfassend methanotrophe Bakterien, optional in Kombination mit einer oder mehreren Spezies heterotropher Bakterien.

2. Verfahren nach Anspruch 1, ferner umfassend einen Trocknungsschritt des resultierenden, homogenisierten Produkts.

3. Verfahren nach Anspruch 1, einem Einzellerproteinmaterial verbesserte funktionelle Eigenschaften zu verleihen, wobei das besagte Verfahren die folgenden Schritte umfasst:
(a) Herstellung eines wässerigen Schlamms von dem Einzellerproteinmaterial, welches eine mikrobielle Kultur ist, umfassend methanotrophe Bakterien, optional in Kombination mit einer oder mehreren Spezies heterotropher Bakterien;
(b) Unterwerfung des Schlamms einem Gesamtdruckabfall in dem Bereich von 40 MPa bis 120 MPa (400 bis 1200 bar), wobei ein homogenisiertes Produkt hergestellt wird; und
(c) optionales Trocknen des homogenisierten Produkts.

4. Verfahren nach einem der Ansprüche 1 bis 3, worin die Homogenisierung bei einer Temperatur von weniger als 50°C, vorzugsweise von 25 bis 50°C, zum Beispiel von 25 bis 35°C durchgeführt wird.

5. Verfahren nach einem der Ansprüche 1 bis 4, worin der besagte Druckabfall stufenweise ist.

6. Verfahren nach Anspruch 5, worin der besagte Druckabfall als ein zweistufiger Prozess durchgeführt wird, umfassend einen ersten und einen zweiten Schritt, wobei der Druckabfall in dem zweiten Schritt weniger als ein fünftel des Gesamtdruckabfalls repräsentiert.

7. Verfahren nach einem der vorhergegangenen Ansprüche, worin das resultierende, homogenisierte Produkt mittels Sprühtrocknung getrocknet wird.

8. Verfahren nach einem der vorhergegangenen Ansprüche, worin vor der Sprühtrocknung das homogenisierte Produkt in einen Speicherbehälter bei einer Temperatur von weniger als 20°C und einem pH von weniger als etwa 6,5 gehalten wird.

9. Verfahren nach einem der vorhergegangenen Ansprüche, worin die besagte mikrobielle Kultur methanotrophe Bakterien und heterotrophe Bakterien umfasst.

10. Verfahren nach Anspruch 9, worin die besagte mikrobielle Kultur eine Kombination aus dem methanotrophen Bakterium *Methylococcus capsulatus* (Bath) (Stamm NCIMB 11132) und den heterotrophen Bakterien *Alcaligenes acidovorans* DB3 (Stamm NCIMB 13287) und *Bacillus firmus DB* 5 (Stamm NCIMB 13289) umfasst, optional in Kombination mit *Bacillus brevis* DB4 (Stamm NCIMB 13288).

11. Verfahren nach einem der vorhergegangenen Ansprüche, worin das besagte Einzellermaterial aus einer Fermentation mit Kohlenwasserstofffraktionen oder Erdgas stammt.

12. Homogenisiertes Einzellerproteinmaterial, das durch ein Verfahren nach Anspruch 9 oder 10 erhalten wird.

13. Homogenisiertes Einzellerproteinmaterial, umfassend eine Kombination aus dem methanotrophen Bakterium *Methylococcus capsulatus* (Bath) (Stamm NCIMB 11132) und den heterotrophen Bakterien *Alcaligenes acidovorans* DB3 (Stamm NCIMB 13287) und *Bacillus firmus* DB 5 (Stamm NCIMB 13289), optional in Kombination mit *Bacillus brevis* DB4 (Stamm NCIMB 13288).

14. Produkt oder Additiv mit Lebensmittelunbedenklichkeit, zum Beispiel Tierfutter oder Tiernahrung, umfassend ein Einzellermaterial nach einem der Ansprüche 12 oder 13.

15. Tiernahrung, umfassend Brocken eines Einzellermaterials nach einem der Ansprüche 12 oder 13.

16. Produkt oder Additiv mit Lebensmittelunbedenklichkeit, zum Beispiel Tierfutter oder Tiernahrung, umfassend ein homogenisiertes Eizizellerproteinmaterial, welches durch ein Verfahren nach einem der Ansprüche 1 bis 11 erhalten wird, zusammen mit einem Produkt mit Lebensmittelunbedenklichkeit.

17. Verwendung eines homogenisierten Einzellerproteinmaterials, welches durch ein Verfahren nach einem der Ansprüche 1 bis 11 erhalten wird, als ein Geliermittel, Emulgator oder als ein Öl- oder Wasserbinder bei der Hestellung eines Nahrungsmittel- oder Futterprodukts.

## Revendications

1. Procédé pour homogénéiser une matière protéique unicellulaire, ledit procédé comprenant l'étape d'homogénéisation d'une suspension aqueuse de la protéine unicellulaire en la soumettant à une chute de pression totale dans la plage de 40 MPa à 120 MPa (400 à 1200 bar), où ladite matière protéique unicellulaire est une culture microbienne comprenant des bactéries méthanotrophes éventuellement en combinaison avec une ou plusieurs espèces de bactéries hétérotrophes.

2. Procédé selon la revendication 1 comprenant en outre l'étape de séchage du produit homogénéisé résultant.

3. Procédé selon la revendication 1 pour conférer des propriétés fonctionnelles améliorées à une matière protéique unicellulaire, ledit procédé comprenant les étapes suivantes :
(a) préparation d'une suspension aqueuse d'une matière unicellulaire qui est une culture microbienne comprenant les bactéries méthanotrophes éventuellement en combinaison avec une ou plusieurs espèces de bactéries hétérotrophes ;
(b) exposition de la suspension à une chute de pression totale de 40 MPa à 120 MPa (400 à 1200 bar), pour produire un produit homogénéisé ; et
(c) éventuellement séchage du produit homogénéisé.

4. Procédé selon l'une quelconque des revendications 1 à 3 où l'homogénéisation est réalisée à une température inférieure à 50°C, de préférence de 25 à 50°C, par exemple de 25 à 35°C.

5. Procédé selon l'une quelconque des revendications 1 à 4 où ladite chute de pression est par étapes.

6. Procédé selon la revendication 5 où ladite chute de pression est réalisée sous forme d'un procédé en deux étapes comprenant des première et seconde étapes où la chute de pression dans la seconde étape représente moins d'un cinquième de la chute de pression totale.

7. Procédé selon l'une quelconque des revendications précédentes où le produit homogénéisé résultant est séché par séchage par dispersion.

8. procédé selon l'une quelconque des revendications précédentes où, avant le séchage par dispersion, le produit homogénéisé est maintenu dans un réservoir de stockage à une température inférieure à 20°C et un pH inférieur à environ 6,5.

9. Procédé selon l'une quelconque des revendications précédentes où ladite culture microbienne comprend des bactéries méthanotrophes et hétérotrophes.

10. Procédé selon la revendication 9 où ladite culture microbienne comprend une combinaison de la bactérie méthanotrophe *Methylococcus capsulatus* (Bath) (souche NCIMB 11132), et des bactéries hétérotrophes *Alcaligenes acidovorans* DB3 (souche NCIMB 13287) et *Bacillus firmus* DB 5 (souche NCIMB 13289), éventuellement en combinaison avec *Bacillus brevis* DB4 (souche NCIMB 13288).

11. Procédé selon l'une quelconque des revendications précédentes où ladite matière unicellulaire est issue de la fermentation sur des fractions hydrocarbonées ou sur du gaz naturel.

12. Matière protéique unicellulaire homogénéisée pouvant être obtenue par un procédé selon la revendication 9 ou la revendication 10.

13. Matière protéique unicellulaire homogénéisée comprenant une combinaison de la bactérie méthanotrophe *Methylococcus capsulatus* (Bath) (souche NCIMB 11132), et des bactéries hétérotrophes *Alcaligenes acidovorans* DB3 (souche NCIMB 13287) et *Bacillus firmus* DB 5 (souche NCIMB 13289), éventuellement en combinaison avec *Bacillus brevis* DB4 (souche NCIMB 13288).

14. Produit ou additif de qualité alimentaire, par exempte aliment pour animaux ou aliment pour animaux familiers, comprenant une matière unicellulaire selon la revendication 12 ou la revendication 13.

15. Aliment pour animaux familiers comprenant des blocs d'une matière unicellulaire selon la revedication 12 ou la revendication 13.

16. Produit ou additif de qualité alimentaire, par exemple aliment pour animaux ou aliment pour animaux familiers, comprenant une matière protéique unicellulaire homogénéisée pouvant être obtenue par un procédé selon l'une quelconque des revendications 1 à 11, ainsi qu'un produit de qualité alimentaire.

17. Utilisation d'une matière protéique unicellulaire homogénéisée pouvant être obtenue par un procédé selon l'une quelconque des revendications 1 à 11 comme agent gélifiant, émulsifiant ou liant l'huile ou l'eau dans la préparation d'un produit alimentaire ou alimentaire pour animaux.
